Europäisches Patentamt

European Patent Office

Office européen des brevets

(19)

(11) Veröffentlichungsnummer: **0 118 066**

**A1**

(12) # EUROPÄISCHE PATENTANMELDUNG

(21) Anmeldenummer: **84101670.2**

(22) Anmeldetag: **17.02.84**

(51) Int. Cl.³: **A 61 F 5/44**
**A 61 B 5/00**

(30) Priorität: **18.02.83 CH 935/83**
**29.03.83 CH 1731/83**

(43) Veröffentlichungstag der Anmeldung:
**12.09.84 Patentblatt 84/37**

(84) Benannte Vertragsstaaten:
**AT BE CH DE FR GB IT LI LU NL SE**

(71) Anmelder: **Sterimed Gesellschaft für medizinischen Bedarf mbH**
**Fasanerieweg 15**
**D-6600 Saarbrücken(DE)**

(72) Erfinder: **Nagorski, Klaus-Peter**
**Untere Bergerheide 57**
**D-5600 Wuppertal(DE)**

(72) Erfinder: **Kramann, Bernhard, Prof. Dr.**
**Preysingstrasse 11**
**D-8000 München 51(DE)**

(74) Vertreter: **Suchy, Herbert, Dr.**
**Byk Gulden Lomberg Chemische Fabrik GmbH**
**Byk-Gulden-Strasse 2 Postfach 6500**
**D-7750 Konstanz(DE)**

(54) Sekretbeutel.

(57) Es wird ein Sekretbeutel für medizinische Zwecke mit Einlaufröhrchen beschrieben, dessen Beutel (1) eine oder mehrere Meßkammern (4, 4a, 4b ...) mit Feinmeßgraduierungen (7, 7a, 7b, ...) aufweist, wobei diese Meßkammern so angeordnet sind, daß sie bei Sekretzulauf nacheinander beschickt werden. Der Sekretzufluß pro Zeiteinheit kann so ohne besondere zusätzliche Einrichtungen einfach bestimmt werden.

EP 0 118 066 A1

./...

Fig.1

S29 EP                    -1-                    0118066
2/84

Sekretbeutel

## Technisches Gebiet

Die Erfindung bezieht sich auf einen Sekretbeutel, insbesondere Urinbeutel, mit Sekret- bzw. Urinmengenmeßeinrichtung.

## Stand der Technik

In der Krankenpflege, insbesondere bei der Intensivpflege und während Operationen, besteht häufig das Problem, die Menge an abgesonderten Sekreten pro Zeiteinheit laufend zu überwachen. Beispielsweise gibt die pro Zeiteinheit abgegebene Urinmenge wichtige Aufschlüsse über die Funktion von Nieren und Herz.

Für ganz grobe Messungen, z.B. Messung der Tagesurinmenge, kann die auf den üblichen Sekretbeuteln angebrachte Graduierung herangezogen werden. Für Feinmessungen, z.B. Stundenurinmessung, ist die Graduierung naturgemäß zu ungenau. Man schaltet deshalb zwischen Blasenkatheter und Auffangbeutel ein sogenanntes Urimeter dazwischen. Weniger arbeitesaufwendig und aus hygienischer Sicht zu bevorzugen sind Sekretbeutelsysteme mit integrierter Volumenfeinmeßmöglichkeit.

So wird in der FR-OS 2 178 022 ein Sammelbeutel vorgeschlagen, der im oberen Teil ein konisches Meßabteil aufweist, das mittels einer Klemme gegenüber dem unteren Hauptteil abgeschlossen ist.

In der US-PS 4 095 589 wird ein flexibler Beutel vorgeschlagen, der mit einer halbstarren Meßkammer verbunden ist, wobei nach der Messung der Inhalt der Meßkammer in den Beutel gekippt werden kann.

In der EP-OS 42 499 wird ein Beutel vorgeschlagen, dessen oberes Ende sich konisch verjüngt und bei Gebrauch gegen den Hauptbeutel nach unten gebogen wird. Das Meßteil wird durch einfaches Hochheben in den Hauptbeutel entleert.

Darstellung der Erfindung

Die Aufgabe der vorliegenden Erfindung kann darin gesehen werden, derartige Sekretbeutelsysteme mit integriertem Meßteil im Hinblick auf ihre Fertigung und Handhabung zu vereinfachen.

Diese Aufgabe wird erfindungsgemäß gelöst durch einen Sekretbeutel für medizinische Zwecke mit Einlaufröhrchen, dadurch gekennzeichnet, daß der Sekretbeutel eine oder mehrere Meßkammern mit Feinmeßgraduierung aufweist, wobei die Meßkammern so angeordnet sind, daß sie bei Sekretzulauf nacheinander beschickt werden.

Die Herstellung der erfindungsgemäßen Sekretbeutel ist denkbar einfach und entspricht der bei handelsüblichen Sekretbeuteln. Die Kammerbegrenzungen innerhalb des Beutels werden als Schweißnähte ausgebildet, wie sie auch bisher bei der Sekretbeutelherstellung bereits üblich sind. Gleichermaßen können die Kammerbegrenzungen auch als eingeschweißte Stege ausgebildet werden. Weiterhin können die Kammerbegrenzungen durch von außen an den Sekretbeuteln angebrachte Klemmleisten erzeugt werden.

S29 EP                          -3-                        0118066

Weitere Gegenstände der Erfindung sind die in den Patentansprüchen angegebenen Ausführungsformen.

Zum näheren Verständnis soll die Erfindung anhand der Fig.
1 bis 7 näher erläutert werden.

Fig. 1 zeigt einen Sekretbeutel mit zwei Meßkammern.

Fig. 2 zeigt einen Sekretbeutel mit einer Meßkammer mit
        Ablaßventil.

Fig. 3 und 4 zeigen Sekretbeutel mit kaskadenförmig
        angeordneten Meßkammern.

Fig. 5 zeigt einen Sekretbeutel mit parallel zur Mittelachse des Beutels verlaufenden Meßkammern.

Fig. 6 zeigt einen Sekretbeutel mit einer Meßkammer in
        perspektivischer Ansicht.

Fig. 7 zeigt eine als Kammerbegrenzung wirkende Klemmleiste
        im Querschnitt nach Linie VII - VII der Fig. 6.

Gleiche Bezugszeichen beziehen sich in den verschiedenen
Figuren jeweils auf das entsprechende Teil.

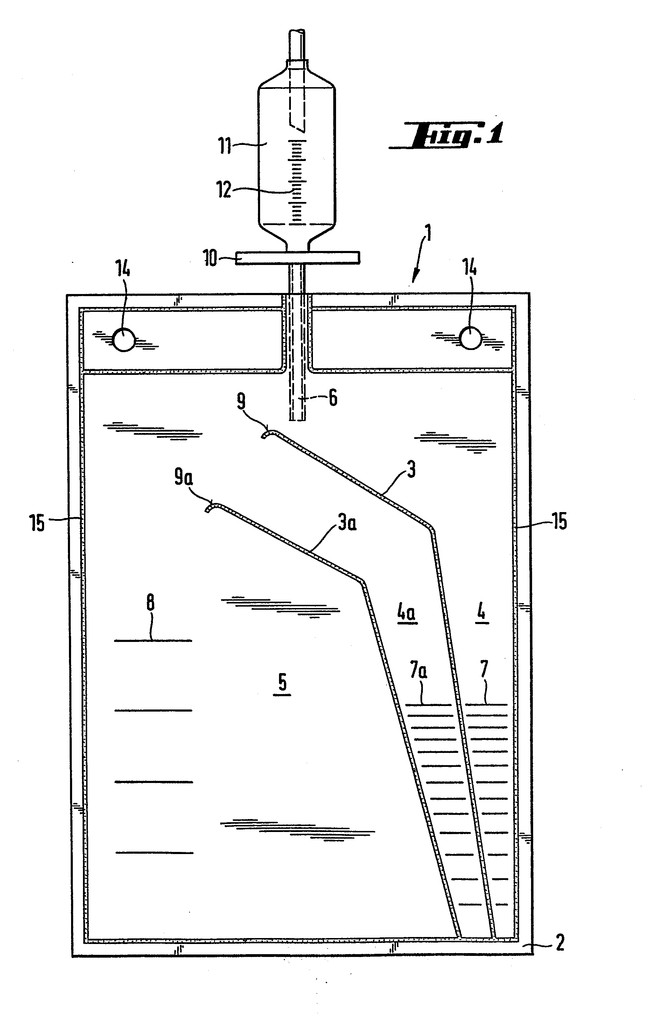

Fig. 1 zeigt einen Sekretbeutel 1, der durch schräg von
oben nach unten zu einer unteren Beutelecke 2 hin verlaufende Kammerbegrenzungen 3, 3a in Meßkammern 4, 4a und eine
Sammelkammer 5 unterteilt wird. Die Meßkammern 4, 4a weisen
Feingraduierungen 7, 7a und die Sammelkammer eine Grobgraduierung 8 auf. Die Kammerbegrenzung 3 endet unterhalb des
unteren Endes des Einlaufröhrchens 6, so daß bei Zutropfen
von Sekret sich zuerst die Meßkammer 4 füllt.

Die Meßkammern 4, 4a können an ihrem unteren Ende konisch verjüngt ausgeführt werden, womit die Ablesegenauigkeit für die ersten in die jeweilige Meßkammer fließenden Sekretmengen erhöht wird.

Sobald Meßkammer 4 gefüllt ist, tropft das Sekret entsprechend der durch das Einlaufröhrchen 6 hinzukommenden Menge über die Abtropfkante 9 in die Meßkammer 4a. Auch diese Kammer besitzt eine Feingraduierung 7a, so daß über mehrere Stunden ein lückenloses Protokoll der Sekretflußrate dokumentiert werden kann. Bei Normalisierung des Sekretflusses und bei Füllung der Meßkammer 4a läuft das Sekret dann über die Abtropfkante 9a in die Sammelkammer 5. Auch diese weist eine Graduierung 8 auf. Zusätzlich ist es möglich, durch Absperrung der Tropfkammer 11, die ebenfalls eine Graduierung 12 aufweist, mittels einer Schiebeklemme 10 eine Mikromessung kurzzeitig, z.B. während der Visite, vorzunehmen. Der Sekretbeutel 1 mit der aufgesetzten Tropfkammer 11 und der dazwischenliegenden Schiebeklemme 10 hat weiterhin den Vorteil, daß beim Wechseln des Sekretbeutels 1 die Tropfkammer 11 verschlossen werden kann, so daß eine aufsteigende bakterielle Kontamination vermieden wird.

Außerdem kann der beschriebene Sekretbeutel gemäß Fig. 1 pro Kammer ein Ablaßventil besitzen (in Fig. 1 nicht gezeigt). Das beschriebene Tropfkammersystem und die Ablaßventile sind ebenfalls bei den nachstehenden Ausführungen verwendbar. Die Aufhängung der Sekretbeutel 1 erfolgt an üblichen Sekretbeutelhaltern beispielsweise mittels Aufhängeösen 14.

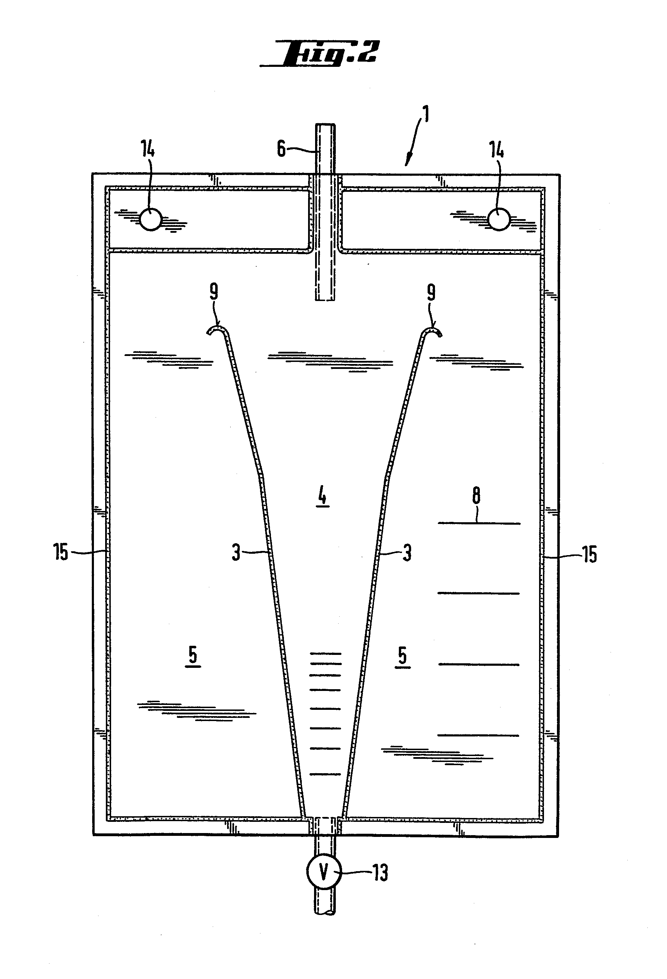

Fig. 2 zeigt einen Sekretbeutel 1, der mittig eine trichterförmige Meßkammer 4 aufweist. Über die Verjüngung nach unten zum Ablaßhahn 13 ist es möglich, kleine Flüssigkeitsmengen pro Zeiteinheit zu bestimmen. Bei Erreichen des Meßkammervolumens läuft das Sekret über die Überlaufkanten 9 in den Rest des vorhandenen Sekretbeutels.

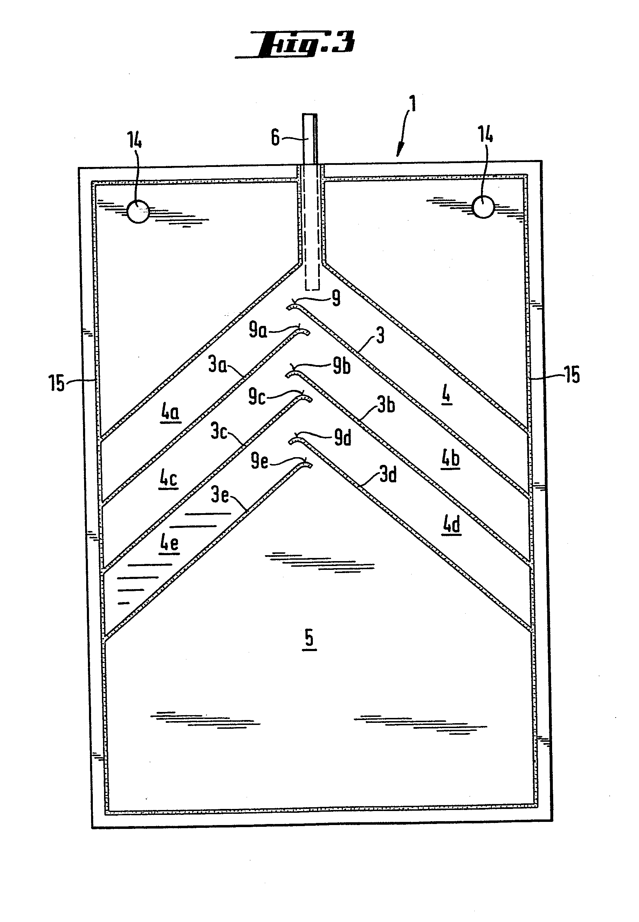

Fig. 3 zeigt einen Sekretbeutel 1, bei dem die Kammerbegrenzungen 3, 3a, 3b, 3c usw. kammartig von den beiden seitlichen Begrenzungen 15 des Sekretbeutels 1 schräg nach oben bis knapp über die Beutelmittellinie laufen, so daß die Meßkammern 4, 4a, 4b, 4c usw. sich kaskadenartig nacheinander von oben nach unten abwechselnd links und rechts füllen.

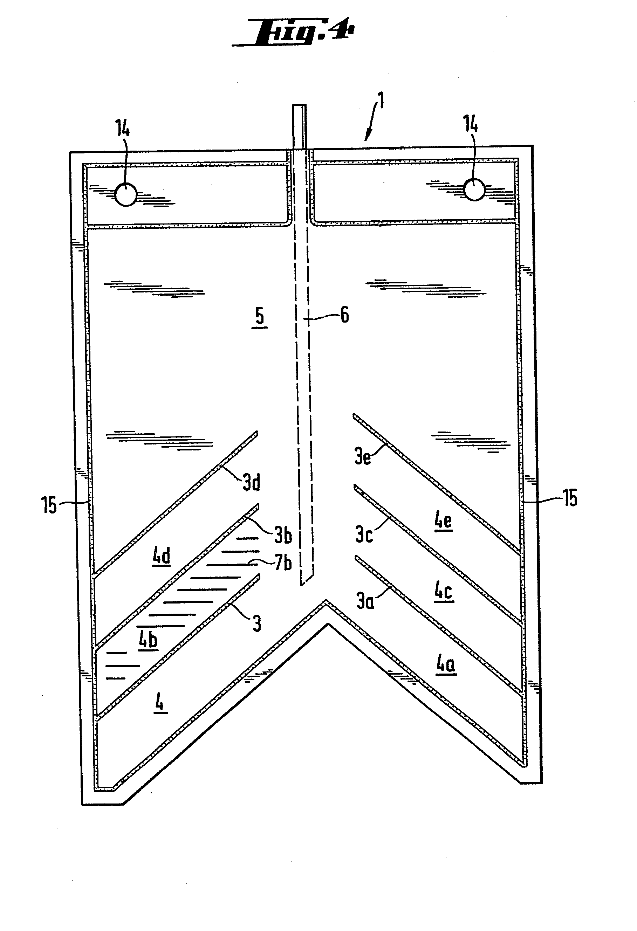

Fig. 4 zeigt einen Sekretbeutel 1 mit einem annähernd bis an das untere Ende des Sekretbeutels 1 reichenden Einlaufröhrchen 6, bei dem die Kammerbegrenzungen 3, 3a, 3b, 3c usw. so angeordnet sind, daß sich die Meßkammern 4, 4a, 4b, 4c usw. kaskadenartig von unten nach oben abwechselnd links und rechts füllen.

Bei den Ausführungsformen gemäß Fig. 3 und 4 kann gewünschtenfalls der gesamte Sekretbeutel durch Meßkammern ausgefüllt sein, so daß keine eigentliche Sammelkammer 5 vorgesehen wird.

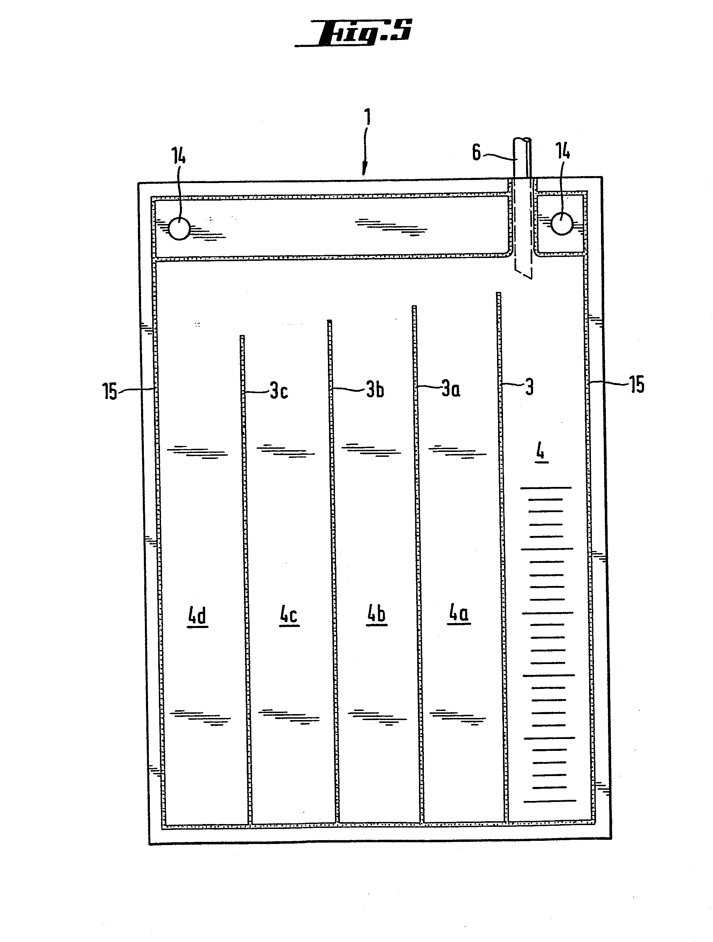

Fig. 5 zeigt einen Sekretbeutel 1 mit parallel zu den seitlichen Begrenzungen 15 verlaufenden Meßkammern 4a, 4b, 4c und 4d, wobei das Einlaufröhrchen 6 oberhalb der an eine seitliche Begrenzung 15 des Sekretbeutels 1 angrenzenden Meßkammer 4 angeordnet ist.

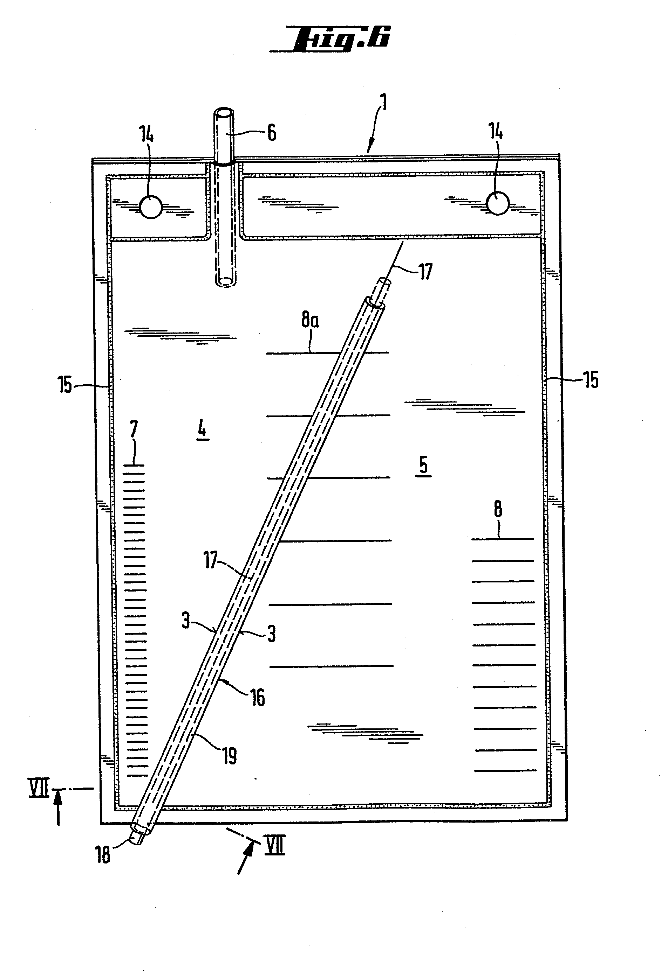

Fig. 6 zeigt einen Sekretbeutel 1, bei dem die Kammerbegrenzung 3 durch eine Klemmleiste 16 gebildet ist. Die Meßkammer 4 weist eine Feinmeßgraduierung 7 und die Sammelkammer 5 eine Grobmeßgraduierung 8 auf. Sobald keine Feinmessung mehr benötigt wird, kann die Klemmleiste 16 entfernt werden. Durch den Wegfall der Kammerbegrenzung 3 steht damit für die Sekretaufnahme ein Volumen zur Verfügung, das grösser ist als die Summe der Volumina der Meßkammer 4 und der Sammelkammer 5 und dem Volumen eines normalen Sekretbeutels ohne Kammerbegrenzungen entspricht. Zur Anzeige der Füllmenge bei Verwendung des Sekretbeutels 1 ohne Kammerbegrenzung 3 dient eine weitere Grobmeßgraduierung 8a. Der Sekretbeutel weist eine Markierung 17 auf, entlang der die Klemmleiste 16 anzubringen ist.

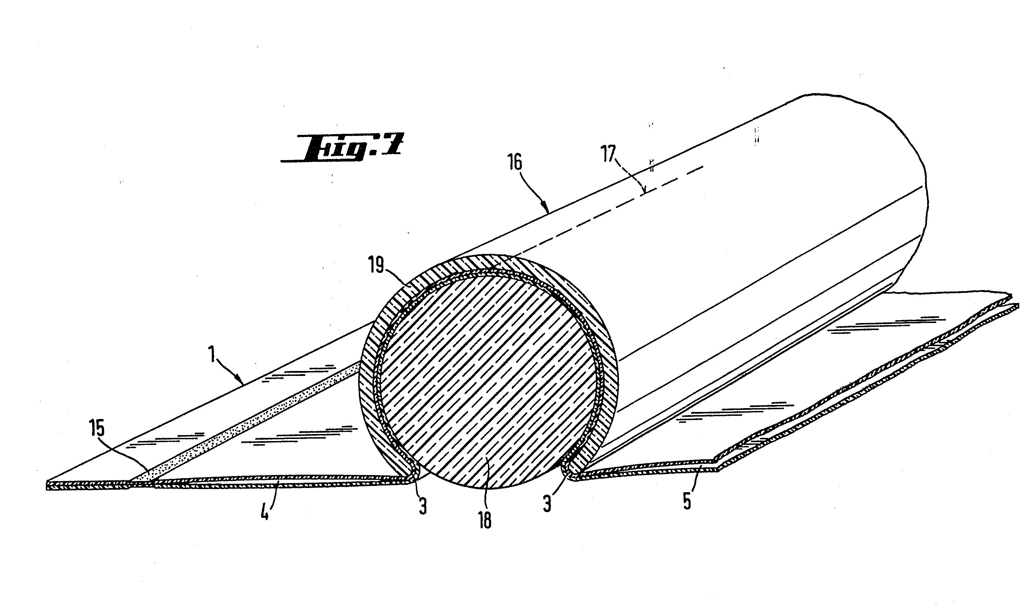

Fig. 7 zeigt schematisch eine Ausführungsform einer Klemmleiste 16 an, die aus einem Rundstab 18 und einer Klemmhülse 19 besteht.

Das Anbringen der Klemmleiste 16 kann erfolgen, indem der Sekretbeutel 1 entlang seiner Markierung 17 (Fig. 6) über den Rundstab 18 geschlagen wird und mittels der Klemmhülse 19 zwischen dieser und dem Rundstab 18 entlang der Markierung 17 eingeklemmt wird. Durch einfaches Abziehen der Klemmhülse 19 läßt sich so bei Bedarf die Kammerbegrenzung 3 aufheben.

Die beschriebenen Sekretbeutelsysteme können sowohl mit Lippen- als auch mit Kugelventilen gegen ein Überlaufen gesichert werden.

<u>P a t e n t a n s p r ü c h e</u>

1.      Sekretbeutel für medizinische Zwecke mit Einlaufröhrchen, dadurch gekennzeichnet, daß der Beutel (1) eine
oder mehrere Meßkammern (4, 4a, 4b ...) mit Feinmeßgraduierungen (7, 7a, 7b, ...) aufweist, wobei diese Meßkammern so
angeordnet sind, daß sie bei Sekretzulauf nacheinander beschickt werden.

2.      Sekretbeutel nach Anspruch 1, dadurch gekennzeichnet, daß die Meßkammern (4, 4a, 4b ...) sich nach unten konisch verjüngen.

3.      Sekretbeutel nach Anspruch 1, dadurch gekennzeichnet, daß die Meßkammern (4, 4a, 4b ...) im wesentlichen parallel zu einer seitlichen Begrenzung (15) des Sekretbeutels (1) verlaufen.

4.      Sekretbeutel nach einem der Ansprüche 1 bis 3, dadurch gekennzeichnet, daß die Meßkammern (4, 4a, 4b ...) am
unteren Ende ein Ablaßventil (13) aufweisen.

5.      Sekretbeutel nach einem der Ansprüche 1 bis 3, dadurch gekennzeichnet, daß die Meßkammern (4, 4a, 4b ...)
von der Beutelmittellinie schräg nach unten zu den Begrenzungen (15) des Sekretbeutels (1) laufen.

6.      Sekretbeutel nach einem der Ansprüche 1 bis 5, dadurch gekennzeichnet, daß die Kammerbegrenzungen (3, 3a, 3b
...) durch Schweißnähte oder eingeschweißte Stege gebildet
werden.

7. Sekretbeutel nach einem der Ansprüche 1 bis 5, dadurch gekennzeichnet, daß die Kammerbegrenzungen (3, 3a, 3b ...) durch Klemmleisten (16) gebildet werden.

8. Sekretbeutel nach Anspruch 6 oder 7, dadurch gekennzeichnet, daß der gesamte Innenraum des Sekretbeutels in Meßkammern (4, 4a, 4b ...) aufgeteilt ist.

Fig.1

**Fig.2**

# Fig.3

Fig.4

## Fig.5

Fig:6

Fig.7

Europäisches
Patentamt

**EUROPÄISCHER RECHERCHENBERICHT**

0118066
Nummer der Anmeldung

EP 84 10 1670

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-3 831 453 (McWHORTER)<br>* Abbildungen; Spalte 1, Zeile 52 - Spalte 3, Zeile 18 * | 1-8 | A 61 F 5/44<br>A 61 B 5/00 |
| | --- | | |
| X | EP-A-0 008 450 (INTERMEDICAT)<br><br>* Abbildungen; Zusammenfassung * | 1,2,4, 6,8 | |
| | --- | | |
| A | GB-A-1 506 457 (FRANKLIN)<br>* Abbildungen; Seite 2, Zeilen 84-94 * | 7 | |
| | ----- | | |

RECHERCHIERTE
SACHGEBIETE (Int. Cl. ³)

A 61 F
A 61 B

Der vorliegende Recherchenbericht wurde für alle Patentansprüche erstellt.

| Recherchenort<br>DEN HAAG | Abschlußdatum der Recherche<br>28-05-1984 | Prüfer<br>STEENBAKKER J. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82